# EUROPEAN PATENT APPLICATION

(11) **EP 3 363 343 A1**
(43) Date of publication of application: **22.08.2018**
(21) Application number: 16855517.5
(22) Date of filing: 14.10.2016
(51) Int. Cl.: A61B 1/00, G02B 23/24, G03B 17/56

(54) **DRAPE FOR CAMERA**

(30) Priority: 16.10.2015 JP 2015204890
(71) Applicant: Okura Industrial Co., Ltd., Marugame-shi Kagawa 763-8508 (JP)
(72) Inventor: YAMASAKI, Junji, Marugame-shi Kagawa 763-8508 (JP); TAKAISHI, Yoshiki, Marugame-shi Kagawa 763-8508 (JP); ORIHARA, Masanao, Marugame-shi Kagawa 763-8508 (JP); AKUNE, Yasuhiro, Marugame-shi Kagawa 763-8508 (JP)
(74) Representative: Mai Dörr Besier European Patent Attorneys
(86) International application number: PCT/JP2016/080518
(87) International publication number: WO 2017/065264

(57) **Abstract**

A camera drape that allows easy connection between a camera and an endoscope is provided. The camera drape for covering a camera to which an endoscope is connected includes a cylindrical camera drape film (10) having, in the following order from one end (10X) to the other end (10Y) thereof, a main body portion (11); and a distal end portion (12) for holding and accommodating the camera, the distal end portion having a smaller inside diameter than the main body portion.

## Description

### Technical Field

The present invention relates to a camera drape for covering a camera to which an endoscope is to be connected.

### Background Art

In recent years, endoscopic surgeries using an endoscopic device including a camera connected to an endoscope are practiced as minimally invasive medical procedures that impose less burden on patients' bodies. In an endoscopic surgery, the endoscope must be used in sanitary conditions because body fluids or medicinal solutions may adhere to the endoscope during surgery and because the endoscope is inserted into the patient's body. Thus, the endoscope and the camera must be sterilized before surgery to prevent infection. This not only leads to an increase in costs and time but also other problems such as deterioration of the camera caused by sterilization. Thus, to ensure cleanliness of surfaces of the camera, Patent Documents 1 and 2, for example, disclose a camera drape including a cylindrical camera drape film used to cover a camera and a cylindrical receptacle for containing the camera drape film in a folded state.

The camera drape films of the camera drapes of Patent Documents 1 and 2 have a distal end portion formed with a connecting portion for allowing connection between a camera and an endoscope and a proximal end portion fixed in the receptacle. A method of using the camera drape of the Patent Document 1 or 2 is, for example, as follows. A coupling portion of the endoscope is placed at the connecting portion of the distal end portion of the camera drape film. Then, with the endoscope maintained in this state, a coupling portion of the camera is placed at the coupling portion of the endoscope within the camera drape film and is then connected thereto via the camera drape film. Finally, the receptacle is pulled to draw the camera drape film out of the receptacle so that the entire camera is covered with the camera drape film.

### Related Art Document

### Patent Document:

Patent Document 1: US Patent No. 6123080
Patent Document 2: Japanese Unexamined Patent Publication No. JP-A-H07-204211

### Summary of the Invention

### Problems to be Solved by the Invention:

In operating rooms, clean and unclean areas are strictly controlled to prevent infection. When someone handles an unclean thing with clean gloves, he or she can no more handle a clean thing unless he or she changes the gloves to clean ones. If the camera is clean, a single operator can cover the camera with a camera drape (which is naturally clean) without the need to change the gloves to clean ones. However, cameras are usually not sterilized and hence unclean because a sterilization treatment may cause failure or deterioration of cameras. Thus, covering a camera with a camera drape requires the collaboration of two operators, one to hold the camera, which is unclean, and the other to handle the endoscope and the camera drape, which are clean. It is, however, rather difficult for two operators to collaborate to place the endoscope and the camera in position within a camera drape film. This is a serious problem because there is no time to lose in some cases in a special situation like an endoscopic surgery.

It is, therefore, an object of the present invention to provide a camera drape that allows easy connection between a camera and an endoscope. Means for Solving the Problems:

A camera drape according to the present invention for covering a camera to which an endoscope is to be connected includes a cylindrical camera drape film having, in the following order from one end to the other end thereof, a main body portion and a distal end portion for holding and accommodating the camera, the distal end portion having a smaller inside diameter than the main body portion.

With this configuration, because the camera drape film includes the distal end portion with a smaller inside diameter than the main body portion for holding and accommodating the camera, the camera, when introduced into the camera drape film, can be easily placed in position in the distal end portion of the camera drape film. In addition, because the camera can be stably held in positon, the endoscope can be easily connected to the camera.

In the present invention, the camera drape film may further include a guide portion between the main body portion and the distal end portion, the guide portion having an inside diameter that decreases in a direction from the one end to the other end of the camera drape film. With this configuration, because the camera drape film includes the guide portion, which has an inside diameter decreasing in a direction from the one end to the other end of the camera drape film, the camera can be easily guided into the distal end portion of the camera drape film.

In the present invention, the camera includes a camera body, a camera code, and a connecter portion connecting the camera body and the camera code, wherein the distal end portion forms an angle with the guide portion, the angle being generally the same as that formed by the camera body and the connecter portion.

With this configuration, a camera including a camera body and a camera code can be snugly received in the distal end portion and the camera can be easily maintained in position in the camera drape.

In the present invention, the camera drape film has a water vapor permeability of 1000 g/(m² • 24h) or greater when measured at 40°C and 90% RH by a humidity sensor method according to JIS K7129: 2008. With this configuration, the camera drape film can be prevented from fogging during use due to heat from the accommodated camera.

The camera drape of the present invention may further include a connecting member that allows connection between the endoscope and the camera.

The camera drape of the present invention may further include a receptacle for accommodating the camera drape film. With this configuration, unnecessary contamination of the camera drape film itself can be prevented.

### Effect of the Invention

According to the camera drape of the present invention, because a camera can be easily placed and stably maintained in position in the camera drape film, an endoscope can be easily connected to the camera.

### Brief Description of the Drawings

FIG. 1 is a plan view of a camera drape according to one embodiment of the present invention.
FIG. 2 is an enlarged plan view of the camera drape according to one embodiment of the present invention.
FIG. 3 is an enlarged plan view of a camera drape according to another embodiment of the present invention.
FIG. 4 is an enlarged plan view of a camera drape according to another embodiment of the present invention.
FIG. 5 is an enlarged plan view of a camera drape according to another embodiment of the present invention.
FIG. 6 is an enlarged plan view of a camera drape according to another embodiment of the present invention.
FIG. 7 is an enlarged plan view of a camera drape according to another embodiment of the present invention.
FIG. 8 is an enlarged plan view of a camera drape according to another embodiment of the present invention.
FIG. 9 is an enlarged plan view of a camera drape according to another embodiment of the present invention.

### Preferred Embodiments of the Invention

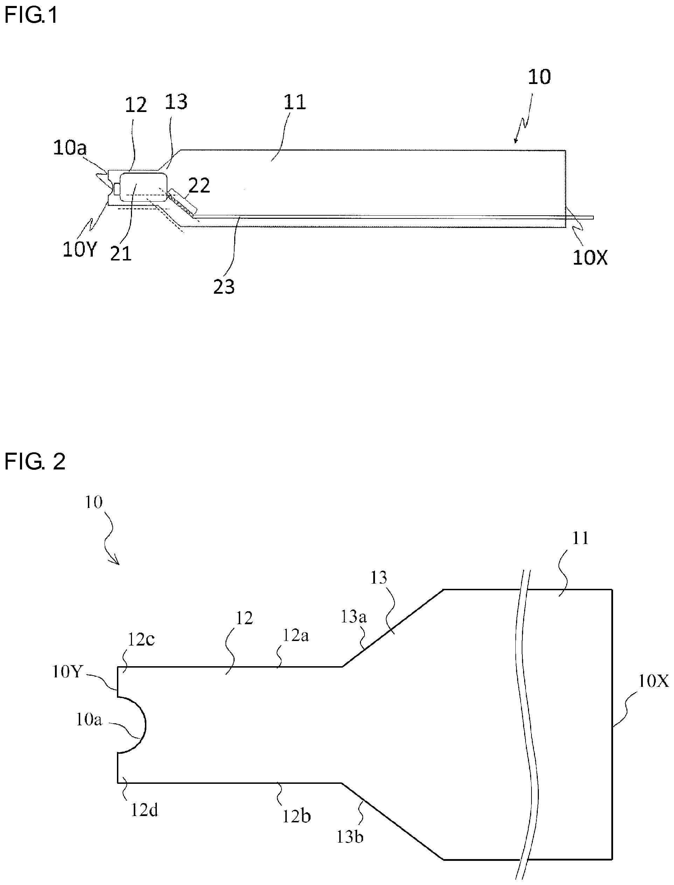

A camera drape according to one embodiment of the present invention will be hereinafter described with reference to the drawings. The camera drape of the present invention includes a camera drape film 10. FIG. 1 is a plan view of the camera drape film 10, and FIG. 2 is an enlarged plan view of the camera drape film 10. As shown in FIG. 1 and FIG. 2, the camera drape film 10, which is used to cover a camera to which an endoscope is to be connected, has a cylindrical shape, and includes, in the following order from one end 10X to the other end 10Y thereof, a main body portion 11, and a distal end portion 12 that has a smaller inside diameter than the main body portion 11 and that is adapted to hold and accommodate the camera. The camera drape film 10 further includes a guide portion 13 between the main body portion 11 and the distal end portion 12. The one end 10X of the camera drape film 10 is open, and the other end 10Y of the camera drape film 10 is closed except for a connecting portion 10a, which is an aperture formed to allow connection between the camera and an endoscope. In some cases, a connecting member may be attached to the connecting portion 10a.

As shown in FIG. 1 and FIG. 2, the main body portion 11 has a rectangular shape, for example, as viewed in a plan view, and has a length of, for example, 2,000 to 5,000 mm from the one end 10X side end to the other end 10Y end thereof. The main body portion 11 preferably has a lay-flat width of 50 to 350 mm, though the lay-flat width is be set based on the shape of the camera used. The term "lay-flat width" is intended to refer to the width between upper and lower ends of the camera drape film 10 as viewed in a plan view when the camera drape film 10 is laid flat on a flat surface. The inside diameter is obtained by dividing the double of the lay-flat width by the circular constant. Thus, the smaller the inside diameter, the smaller is the lay-flat width.

As shown in FIG. 1 and FIG. 2, the distal end portion 12 has a smaller inside diameter than the main body portion 11 and is configured to hold and accommodate a camera. The distal end portion 12 has a rectangular shape, for example, as viewed in a plan view, and has a length of, for example, 100 to 150 mm from the one end 10X side end to the other end 10Y side end thereof and is set in agreement with the length of the camera used. The distal end portion 12 preferably has a length generally the same as the length of the camera used. The distal end portion 12 has a lay-flat width of, for example, 30 to 200 mm and is set in agreement with the size of the camera used. It is preferred that the lay-flat width generally matches to the size of the camera used.

As shown in FIG. 1 and FIG. 2, the guide portion 13 has an inside diameter decreasing from the one end 10X side end toward the other end 10Y side end thereof, and has straight edges 13a and 13b, for example. The guide portion 13 has a length of 50 to 150 mm from the one end 10X side end to the other end 10Y side end thereof. This configuration of the guide portion 13 allows easy introduction of the camera into the distal end portion 12 within the camera drape film 10. In a plan view of the camera drape film 10 shown in FIG. 1 and FIG. 2, the straight line formed by an edge 12a (12b) of the distal end portion 12 and the straight line formed by the edge 13a (13b) of the guide portion 13 preferably form an angle greater than 90 degrees and smaller than 150 degrees, for example. When the camera is constituted of a camera body 21 and a connecter portion 22 that connects the camera body 21 and a camera code 23, the camera body 21 may form an angle with the connecter portion 22 in some cases. In such a case, as shown in FIG. 1, the angle formed by the straight line formed by the edge 12a (12b) of the distal end portion 12 and the straight line formed by the edge 13a (13b) of the guide portion 13 is preferably generally the same as the angle formed by the camera body 21 and the connecter portion 22, more specifically, within ± 5 degrees of the angle formed by the camera body 21 and the connecter portion 22.

The connecting portion 10a is configured to allow connection between the camera and an endoscope. The connecting portion 10a may simply be an opening with a semicircular shape as viewed in a plan view formed near the center of the other end 10Y of the distal end portion 12 as shown in FIG. 1 and FIG. 2. Alternately, a connecting member, for example, may be attached to the connecting portion 10a for ease of connection between the camera and the endoscope.

The camera drape film 10 may be folded in an accordion fashion along its longitudinal direction to reduce the length from the one end 10X to the other end 10Y. Alternately, the camera drape film 10 may be folded back and forth at regular intervals along a direction from the one end 10X to the other end 10Y to make it compact. The intervals are set to 10 to 50 mm, for example. The camera drape film 10 may be simply drawn to one side to make it compact.

The camera drape film 10 is made of a known polymeric resin such as a polyurethane-based resin, a polyethylene-based resin or a polyamide-based resin. Because the inside of the camera drape film 10 may fog due to heat from the accommodated camera during use, the use of a polymeric resin having a water vapor permeability of 1000 g/(m²·24h) or greater when measured at 40°C and 90% RH by a humidity sensor method according to JIS K7129: 2008 is preferred. From the standpoint of providing a close fit for the camera and of improvement of operability of operation buttons of the camera in use, a polyurethane resin is preferred.

The camera drape film 10 has a thickness of 20 to 100 µm, for example.

A method for producing a camera drape film according to one embodiment of the present invention will be next described.

A polymeric resin as described above is formed into a film by an inflation method or a T-die method. When an inflation method is employed, a cylindrical film can be obtained. When a T-die method is employed, a cylindrical film can be obtained by stacking two resulting films and sealing the films along their sides by a known method.

Then, with the one end 10X of the obtained cylindrical film left as it is, heat sealing is carried out by a known method in such a way that the other end 10Y side part of the cylindrical film is formed with the desired shapes of the guide portion 13 and the distal end portion 12 as described above. As a result, the camera drape film 10 including the main body portion 11, the guide portion 13 and the distal end portion 12 can be obtained.

In addition, when the camera drape film is accommodated in a receptacle, the camera drape can be provided.

Description will be next made of how to use the camera drape film 10 according to one embodiment of the present invention. First, a camera drape having a receptacle containing the camera drape film 10 folded at regular intervals is prepared.

Then, a camera is inserted into the camera drape film 10 through an opening of the receptacle, and introduced through the guide portion 13 into the distal end portion 12, where the camera is held and placed in position. This procedure may be carried out by placing the camera with a hand. However, the camera can be almost automatically accommodated and placed in position in the distal end portion 12 when the camera is dropped into the camera drape film 10 held with its distal end portion 12 facing downward. When the camera drape film is made of a soft polymeric resin such as a urethane resin, a camera drape film cannot maintain a cylindrical shape and it is, therefore, not easy to insert the camera close to the distal end portion 12 and place the camera in position unless the distal end portion faces downward even when the camera drape film 10 is contained in a receptacle. Even in such a case, with the camera drape of the present invention, the camera can be placed in position by simply dropping it into the camera drape film 10. Thus, the camera drape of the present invention is particularly useful.

Finally, an endoscope is connected to the camera stably held in the distal end portion 12, and the camera drape film 10 is drawn out of the receptacle to cover the entire camera with the camera drape film 10.

While one embodiment of the present invention is described in the foregoing, specific aspects of the present invention are not limited to the above embodiment. For example, while the camera drape film 10 has the guide portion 13 in the above embodiment shown in FIG. 1 and FIG. 2, the guide portion 13 may be omitted as long as the camera can be easily placed and stably held in position in the distal end portion 12.

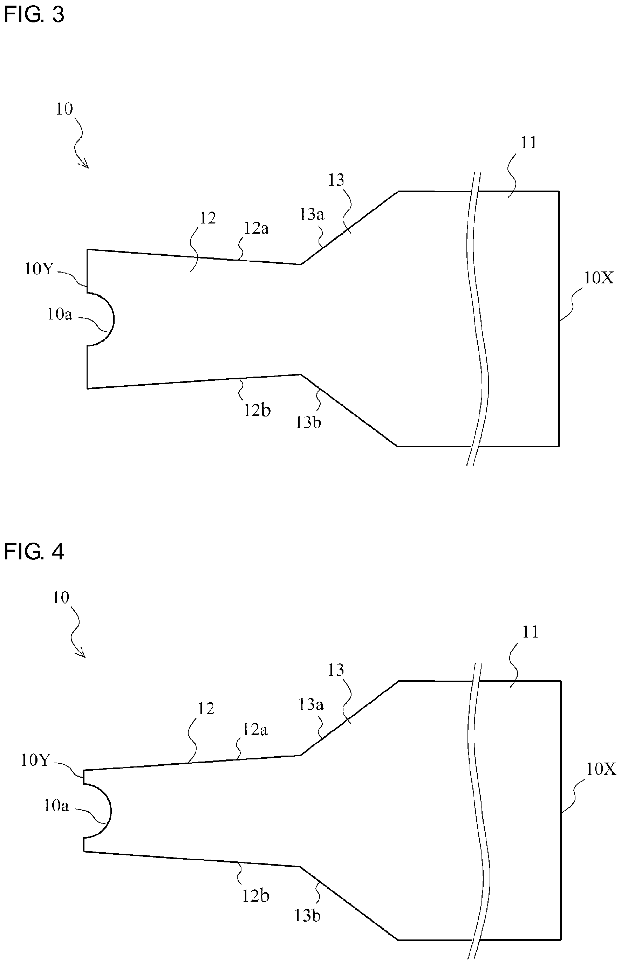

While the distal end portion 12 has a rectangular shape as viewed in a plan view in the above embodiment shown in FIG. 1 and FIG. 2, the shape of the distal end portion 12 is not limited to the rectangular shape. For example, as shown in FIG. 3, the distal end portion 12 may have a trapezoidal shape having an inside diameter increasing in a direction from the one end 10X to the other end 10Y as viewed in a plan view. In contrast to this, as shown in FIG. 4, the distal end portion 12 may have a trapezoidal shape having an inside diameter decreasing in a direction from the one end 10X to the other end 10Y as viewed in a plan view.

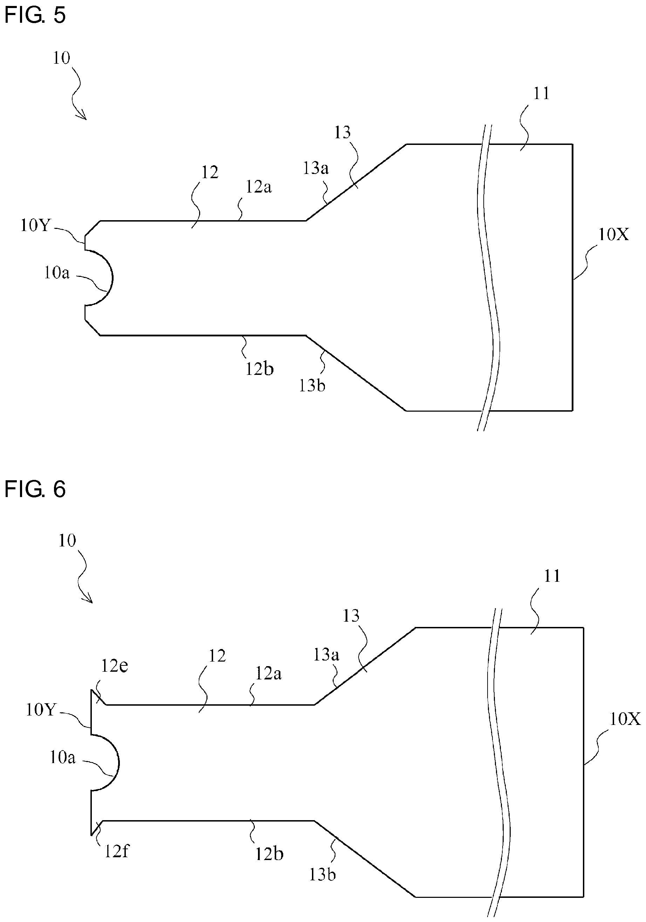

In the above embodiment shown in FIG. 1 and FIG. 2, the distal end portion 12 may have beveled corners 12c and 12d as shown in FIG. 5, for example. Further, as shown in FIG. 6, the distal end portion 12 may have protrusions 12e and 12f, which are triangular as viewed in a plan view, at corners 12c and 12d thereof.

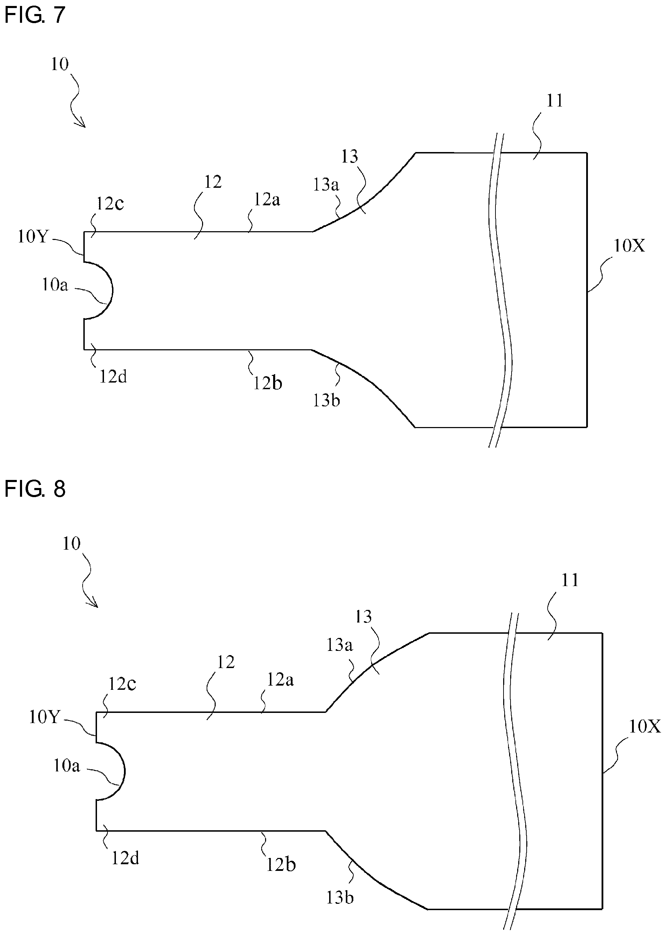

While the guide portion 13 has straight edges 13a and 13b in the above embodiment shown in FIG. 1 and FIG. 2, the edges 13a and 13b may not be necessarily straight. For example, the guide portion 13 may have edges 13a and 13b that are curved to be convex toward the inside of the camera drape film 10 as shown in FIG. 7. In contrast to this, the guide portion 13 may have edges 13a and 13b that are curved to be convex toward the outside of the camera drape film 10 as shown in FIG. 8.

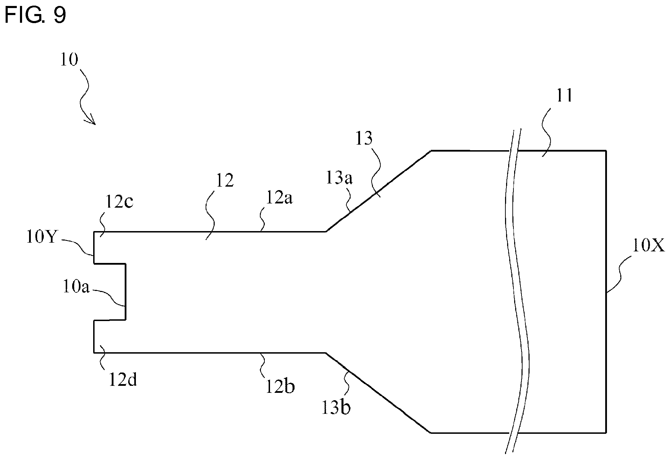

While the connecting portion 10a is formed as an opening with a semicircular shape as viewed in a plan view at the other end 10Y side end of the distal end portion 12 in the above embodiment, the shape of the connecting portion 10a may not be necessarily semicircular. For example, the connecting portion 10a may be formed as an opening with a rectangular shape as viewed in a plan view as shown in FIG. 9.

### Description of Reference Numerals

10: camera drape film
10a: connecting portion
10X: one end
10Y: the other end
11: main body portion
12: distal end portion
12a, 12b: edges of the distal end portion
12c, 12d: corners of the distal end portion
12e, 12f: tabs at the distal end portion
13: guide portion
13a, 13b; edge of the guide portion
21: camera body
22: connecter portion
23: camera code

## Claims

1. A camera drape for covering a camera to which an endoscope is to be connected, comprising a cylindrical camera drape film having, in the following order from one end to the other end thereof,
a main body portion; and
a distal end portion for holding and accommodating the camera, the distal end portion having a smaller inside diameter than the main body portion.

2. The camera drape according to claim 1,
wherein the camera drape film further includes a guide portion between the main body portion and the distal end portion, the guide portion having an inside diameter that decreases in a direction from said one end to said the other end of the camera drape film.

3. The camera drape according to claim 2, wherein the camera includes a camera body, a camera code, and a connecter portion connecting the camera body and the camera code, and wherein the distal end portion forms an angle with the guide portion, said angle being generally the same as that formed by the camera body and the connecter portion.

4. The camera drape according to any one of claims 1 to 3, wherein the camera drape film has a water vapor permeability of 1000 g/(m² • 24h) or greater when measured at 40°C and 90% RH by a humidity sensor method according to JIS K7129: 2008.

5. The camera drape according to any one of claims 1 to 4, further comprising a connecting member that allows connection between the endoscope and the camera.

6. The camera drape according to any one of claims 1 to 5, further comprising a receptacle for accommodating the camera drape film.
